# EUROPEAN PATENT SPECIFICATION

(11) **EP 3 398 499 B1**
(45) Date of publication and mention of the grant of the patent: **20.10.2021**
(21) Application number: 18170244.0
(22) Date of filing: 01.05.2018
(51) Int. Cl.: A61B 1/00, A61B 10/02, A61B 5/00, G16H 30/40, G16H 40/63, G16H 50/20

(54) **INFECTION DETECTION DEVICES**
INFEKTIONSDETEKTIONSVORRICHTUNGEN
DISPOSITIFS DE DÉTECTION D'INFECTIONS

(30) Priority: 02.05.2017 US 201762500206 P; 05.04.2018 US 201815946166
(43) Date of publication of application: 07.11.2018
(73) Proprietor: Covidien LP, Mansfield, MA 02048 (US)
(72) Inventor: SGROI, Anthony, Wallingford, CT 06492 (US)
(74) Representative: Maschio & Soames IP Ltd

(56) References cited:
- WO-A1-00/69332
- WO-A1-2008/137095
- CN-A- 106 178 279
- US-A1- 2011 313 265
- US-A1- 2015 238 128

## Description

This application claims the benefit of and priority to U.S. Provisional Patent Application No. 62/500,206 filed May 2, 2017.

### BACKGROUND

### 1. Technical Field

The present disclosure generally relates to surgical devices, and more particularly, to devices configured for detecting and optionally treating infections at a surgical site.

### 2. Background of Related Art

A surgical site infection is an infection that occurs after surgery in the part of the body where the surgery took place. Surgical site infections can sometimes be superficial infections involving the skin only. Other surgical site infections are more serious and can involve tissues under the skin, organs, or implanted material. There may be a lag between the time the surgery occurs and the onset of signs or symptoms of infection. This may result in an escalation of the condition prior to its detection and the appropriate treatment being instituted.

WO 00/69332 discloses a device according to the preamble of claim 1. Further prior art devices are disclosed in CN 106178279 A, US 2015/238128 and US 2011/3132665.

### SUMMARY

Devices in accordance with the present disclosure provide for minimally invasive, real-time infection detection and, optionally, treatment. The invention is as defined in the appended claims.

According to an aspect of the present disclosure, an infection detection device includes a housing having a display configured to output a level of infection at a surgical site, and a probe having a proximal end portion attached to the housing and a distal end portion configured for insertion into a surgical site. A detection device may be integrated along the distal end portion of the probe. The detection device may be configured to measure a level of infection at a surgical site and to alert a user when a predetermined level of infection is detected at a surgical site.

In embodiments, the detection device includes a camera disposed at a distal end portion of the probe. The camera defines a surgical field of view.

In some embodiments, the detection device includes a light source disposed at the distal end portion of the probe. The light source is configured to illuminate the surgical field of view defined by the camera. The light source is configured to treat an infection at a surgical site.

In the invention, the detection device includes a sterile applicator selectively attachable to a distal end portion of the probe. The sterile applicator is configured to retrieve a tissue sample from a surgical site.

In the invention the infection detection device includes a photometer configured to measure a level of infection from a tissue sample retrieved by the sterile applicator.

In some embodiments, the infection detection device includes a computing device that includes a processor and a memory configured to perform at least one operation of the infection detection device.

In certain embodiments, the display is configured for displaying at least one output of the computing device.

In embodiments, the computing device is configured to determine the predetermined level of infection at the surgical site.

In some embodiments, the display is configured for displaying the surgical field of view defined by the camera.

In certain embodiments, a dial is disposed on the housing and operatively connected to the probe. The dial is configured to selectively rotate and articulate the probe relative to the housing.

In embodiments, the light source is configured to radiate ultraviolet light to visualize a bacterial infection at a surgical site.

In some embodiments, the light source is configured to radiate ultraviolet light with a wavelength from about a 260 nanometer to about a 280 nanometer to treat tissue.

In certain embodiments, the distal end portion of the probe includes a recess configured to receive the sterile applicator.

In the invention the photometer includes a reading chamber configured to receive the sterile applicator for testing a level of infection of a tissue sample therewith.

In some embodiments, the infection detection device includes a data interface device configured to interface with an external device. The data interface device is configured to upload data from the computing device onto the external device. The data interface device is configured to download data from the external device onto the computing device.

According to another aspect of the present disclosure, a method of detecting and treating anastomotic leaks is provided, including making an incision in a patient proximate to a target site, inserting a probe into the incision, viewing the target site with a camera and a light source of the probe, determining with the probe whether the target site includes leaks or infections, and treating the target site with the light source.

In embodiments, the method may include retrieving a tissue sample from the target site with a sterile applicator selectively attached to the probe.

In some embodiments, the method may include measuring a level of infection from a tissue sample retrieved by the sterile applicator with a photometer.

In certain embodiments, the method may include determining, with a computing device, when an infection is present at the target site.

In embodiments, the method may include displaying on a display device a surgical field of view defined by the camera.

In some embodiments, treating the target site includes exposing the target site with ultraviolet light from the light source having a wavelength from about a 260 nanometer to about a 280 nanometer.

In certain embodiments, the method includes visualizing the target site under an ultraviolet light from the light source to view bacterial infections at the target site.

In embodiments, the method includes articulating the probe to navigate to the target site.

### BRIEF DESCRIPTION OF DRAWINGS

Various aspects of the present infection detection devices are described below with reference to the drawings, which are incorporated in and constitute a part of this specification, wherein:
FIG. 1 is a perspective view of an illustrative embodiment of an infection detection device according to the present disclosure;
FIG. 2 is a distal end view of a distal end portion of the device of FIG. 1;
FIG. 3 is a side view of the probe of the device of FIG. 1 with an applicator attached thereto;
FIG. 4 is a front, partial, cross-sectional view of the device of FIG. 1 depicting a reading chamber of a photometer receiving the applicator; and
FIG. 5 is an illustration of the device of FIG. 1 in use at a surgical or wound site.

### DETAILED DESCRIPTION

Embodiments of the present infection detection devices will now be described in detail with reference to the drawings, in which like reference numerals designate identical or corresponding elements in each of the several views. As used herein, the term "clinician" refers to a doctor, nurse, or other care provider and may include support personnel. In the following description, well-known functions or constructions are not described in detail to avoid obscuring the present disclosure in unnecessary detail. As used herein, the term "distal," as is conventional, will refer to that portion of the instrument, apparatus, device or component thereof which is farther from the user or closer to the patient while, the term "proximal," will refer to that portion of the instrument, apparatus, device or component thereof which is closer to the user or further from the patient.

The present disclosure relates to an infection detection device. Specifically, the infection detection and treatment device may be a handheld device having a probe configured to perform a surgical or post-surgical inspection at a surgical or wound site. The present devices may be configured to treat the surgical or wound site by, e.g., reducing or eliminating infectious agents at the surgical or wound site.

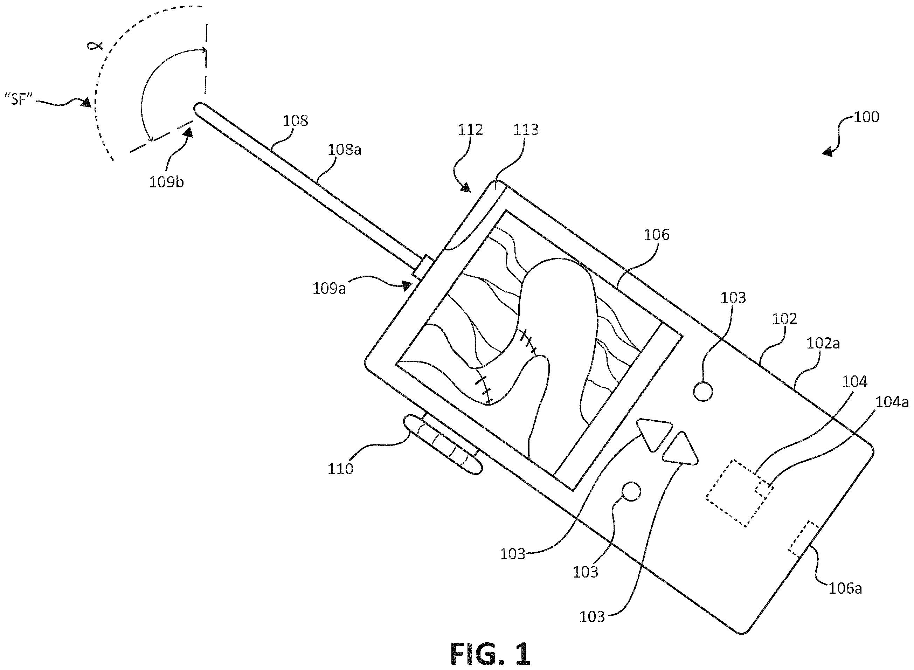

Referring to FIG. 1, an embodiment of an infection detection device 100 in accordance with the present disclosure generally includes a housing 102, a central processing unit 104 (CPU) configured to interface with device 100 and to execute the various operations of the device 100, a display 106, a probe 108 for inspection and/or treatment of a surgical or wound site, a dial 110 for articulating or manipulating probe 108, and a detection device 112 such as, e.g., a luminometer or photometer for analyzing tissue samples from a surgical or wound site. In embodiments, device 100 may be connected to a source of energy to provide power to device 100. Additionally or alternatively, infection 100 may be a portable, battery powered device.

Housing 102 includes a handle portion 102a configured for gripping infection detection device 100, such that device 100 may be configured as a handheld device. Housing 102 includes a plurality of controls 103 configured to receive a user input and to operate various functions of device 100.

CPU 104 may be any type of suitable processor or computer adapted to perform or execute techniques, operations, and/or instructions described herein. For example, CPU 104 may be hardware processors programmed to perform the techniques described herein pursuant to the instructions in firmware, memory, or other storage, or a combination thereof. Similarly, CPU 104 may also be one or more application specific integrated circuits (ASICs), field programmable gate arrays (FPGAs) that are persistently programed to perform the techniques or operations described herein. CPU 104 may also be a digital signal process (DSP), a microprocessor, or any other device that incorporates hard-wired logic or program logic or both to perform the operations or techniques described herein.

CPU 104 may include memory 104a, which may be any type of hardware device used to store data. Memory 104a may be volatile memory, such as random access memory (RAM) (e.g., dynamic random access memory (DRAM), static random access memory (SRAM), etc.). Memory 104a may be non-volatile memory, such as read-only memory (ROM) (e.g., programmable read-only memory (EPROM), electrically erasable programmable read-only memory (EEPROM), non-volatile RAM (NVRAM), etc.). Memory 104a may also be magnetic, optical, or electrical media.

Display 106 may be communicatively connected to CPU 104. Display 106 may be any device (e.g., touch screen, LED, LCD, OLED, PDP, etc.) configured to display at least one output of CPU 104. For example, display 106 may be configured to display images and video of a surgical or wound site, tissue sample data, treatment information, etc. For example, if an infection is present at the surgical or wound site, display 106 may provide a clinician with tissue sample information indicative of an infection or lack thereof, such as "No Infection detected," "Infection detected," "Pass," "Fail," "OK," "RLU," or the like. Display 106 may also display text or graphics recommending a course of treatment, such as, "Apply UV-C light," "Surgery Recommended," etc. In embodiments, the plurality of controls 103 may be digitally displayed on display 106 such that a clinician can touch the plurality of controls 103 on digital display 106 to perform various functions of device 100.

The display 106 may be a liquid crystal display, a plasma display, one or more light emitting diodes, a luminescent display, a multi-color display, an analog display, a passive display, an active display, a "twisted nematic" display, a "super twisted nematic" display, a "dual scan" display, a reflective display, a backlit display, an alpha numeric display, a monochrome display, a "Low Temperature Polysilicon Thin Film Transistor" or LPTS TFT display, or any other display X that indicates a parameter, information or graphics related to infection treatment information of the infection detection and treatment device 100.

Device 100 may include a data interface device 106a configured to interface with an external device, e.g., a tablet, smart device, computer, etc. Data interface device 106a may be configured to upload data from CPU 104 onto an external device. Additionally, data interface device 106a may be configured to download data from an external device onto CPU 104 to change and/or update the programming of CPU 104 (e.g., firmware updates). Data interface device 106a may be a Universal Serial Bus (USB) connector configured to interface with an external device using a USB cable. Additionally or alternatively, data interface device 106a may be configured to receive a USB flash media drive, an SD card, or other removable or non-removable storage medium to download/upload data from CPU 104 and/or memory 104a. Data interface device 106a may include wireless technology (e.g., Bluetooth) configured for receiving and/or transmitting data from and/or to one or more external devices or systems.

Probe 108 includes an elongate tubular body portion 108a having a proximal end portion 109a operatively, communicatively, and/or electrically connected to the housing 102 of device 100, and a distal end portion 109b configured for insertion into, and/or inspection and treatment of, a surgical site, a wound site, or the like, of a patient. Probe 108 may have any suitable length and may be inserted into a patient through any orifice or opening of the patient, or any artificial incision made into the patient. The probe 108 may be formed of any suitable (e.g., non-invasive, biocompatible, etc.), flexible, rigid, semi-flexible, or semi-rigid material, such as plastics, polymers, composites, metals, or the like.

Dial 110 is operably coupled to probe 108 such that rotation (e.g., clockwise or counterclockwise) of dial 110 enables selective rotation, articulation, and/or manipulation of probe 108 relative to housing 102 for navigating a surgical or wound site.

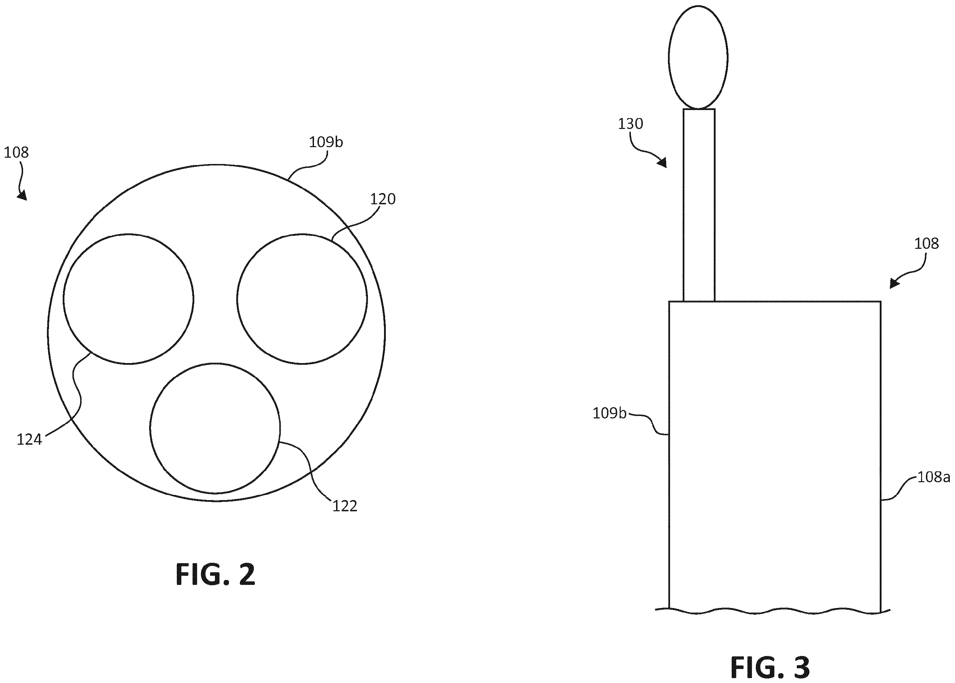

With reference to FIG. 2, probe 108 includes a camera 120 and a light source 122 disposed at the distal end portion 109b thereof. Distal end portion 109b of probe 108 may include a suitable transparent cover (not shown) configured for inhibiting fluids or other contaminants from interfering with the operation of camera 120 and/or light source 122.

Camera 120 defines a surgical field of view "SF" (FIG. 1) and is configured for capturing and recording image and video data of a surgical or wound site. Surgical field of view "SF" may provide a viewing angle "α" up to 360 degrees for navigation of a surgical or wound site. The images and video data of the surgical or wound site are transmitted to and/or displayed on display 106 of device 100 such that a clinician can view or monitor the surgical or wound site in real-time without having to make a large incision into the patient and/or examining the surgical or wound site directly. Image and video data from camera 120 may be recorded into memory 104a of CPU 104 and/or data interface device 106a for later viewing. Image and video data may be transferred to, e.g., a central server, local area network (LAN), wide area network (WAN), and the like, to allow access to the image and video data from remote locations.

Camera 120 may be configured for recording high-definition video (e.g., 1080p, 4K, 8K, etc.) and/or images (e.g., in single or burst mode, high-speed, etc.). Camera 120 may include a wide-angle lens, image stabilizers, image sensors, auto-zoom and omni-directional functionality, or the like. Camera 120 may be a visual-light optical camera, such as a charge-coupled device (CCD), complementary metal-oxide-semiconductor (CMOS), N-type metal-oxide-semiconductor (NMOS), or other suitable camera known in the art. In embodiments, Camera 120 may be an infrared camera or thermographic camera, such as ferroelectric, silicon microbolometer, or uncooled focal plane array (UFPA).

Light source 122 of probe 108 is configured for illuminating surgical field of view "SF" (FIG. 1) defined by camera 120 and is configured to detect and treat infectious agents. For example, light source 122 may radiate or emit ordinary light (e.g., colorless, white light, etc.) to illuminate the surgical field of view "SF," upon which the surgical or wound site can be inspected and/or documented using camera 120. In addition, light source 122 may be configured to radiate ultraviolet (UV) light to illuminate, visualize, and/or treat infectious agents. Probe 108 may be configured to work in combination with a contrasting agent. The contrasting agent can be administered trans-orally into a patient's gastrointestinal tract such that any anastomotic leaks will carry the contrasting agent through the leak and be readily detected by probe 108 of device 100. In other examples, light source 122 can be used to illuminate and visualize infectious agents (e.g., pathogenic bacteria, endotoxins, metabolites, etc.) that would otherwise not be visible under ordinary light. Using UV light, light source 122 causes the infectious agents to fluoresce or exhibit fluorescence, which will expose the infectious agents such that a clinician can readily identify and diagnose the infectious agents. In addition, a clinician can treat the surgical or wound site using UV-C light (e.g., 180 nanometer (nm) to 280nm wavelength) from light source 122 to destroy, kill, or irradiate harmful bacteria, viruses, and other microorganisms. In embodiments, light source 122 is configured to emit ultraviolet light at wavelengths from about 260nm to about 270nm. It should be appreciated that any suitable wavelength configured to target infections or infectious agents may be used, such as, e.g., a specific range of wavelengths for treating the bacteria associated with anastomotic leaks of the GI tract, or a specific range of wavelengths associated with the infections resulting from hernia repair. In embodiments, light source 122 may include one or more light emitting diodes (LED), bulbs, lasers, lamps, microwave generated UV plasma devices, or the like.

With reference to FIGS. 2, 3 and 4, distal end portion 109b of probe 108 may include a recess 124 configured to receive and attach to a sterile applicator or swab 130. The probe 108 with swab 130 attached thereto is configured for insertion into a surgical or wound site to collect tissue cultures or samples. In embodiments, swab 130 may have a push-in, snap-fit, and/or interference fit arrangement with recess 124 of probe 108 such that swab 130 is selectively attached to probe 108. In embodiments, any type of sterile applicator or swab for collecting tissue cultures or samples may be used.

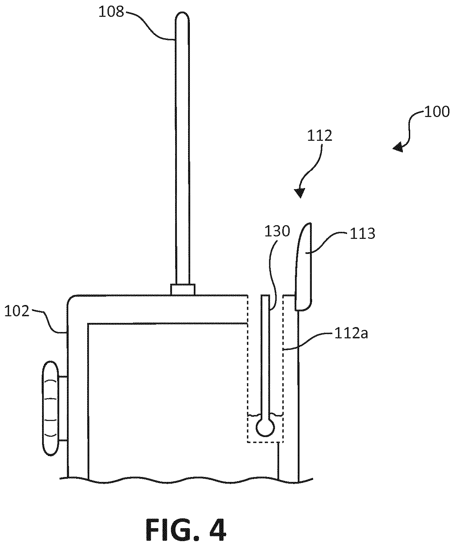

Photometer 112 is communicatively connected to CPU 104. Photometer 112 is configured to perform a real-time tissue culture test, based on a tissue sample collected from a surgical or wound site, to identify the level of infection present and/or the degree of sterility at the surgical or wound site. During operation, a latch 113 of housing 102 is opened to expose a reading chamber 112a of photometer 112. Reading chamber 112a is configured to receive swab 130 therein (FIG. 4) to perform testing of a tissue sample collected by swab 130 and probe 108 from the surgical or wound site.

Photometer 112 may detect or measure levels of adenosine triphosphate (ATP) at a surgical or wound site. Prior to or during insertion of swab 130 into reading chamber 112a of photometer 112, swab 130 may be exposed to or in contact with a reagent (water, luciferase, etc., not shown), such that the ATP present on swab 130, if any, interacts with the reagent to generate light. The amount of light generated is directly proportional to the amount of ATP present, which provides an indication of the total biological contamination level, e.g., the level of sterility or infection at the surgical or wound site. Photometer 112 and CPU 104 cooperate to detect the amount of ATP present to alert the clinician of any infections or pending infections, e.g., at or near a surgical site. Such ATP levels can allow the clinician to determine if intervention is necessary.

The reading may be recorded in relative light units ("RLU"), displayed on display 106, and/or transmitted wirelessly using data interface device 106a to one or more external devices, e.g., to provide a clinician with an assessment of the level of sterility or infection at the surgical or wound site. In embodiments, photometer 112 may include one or more photoresistors, photodiodes, or photomultipliers (not shown) configured to measure light output to approximate the level of sterility or infection at the surgical or wound site.

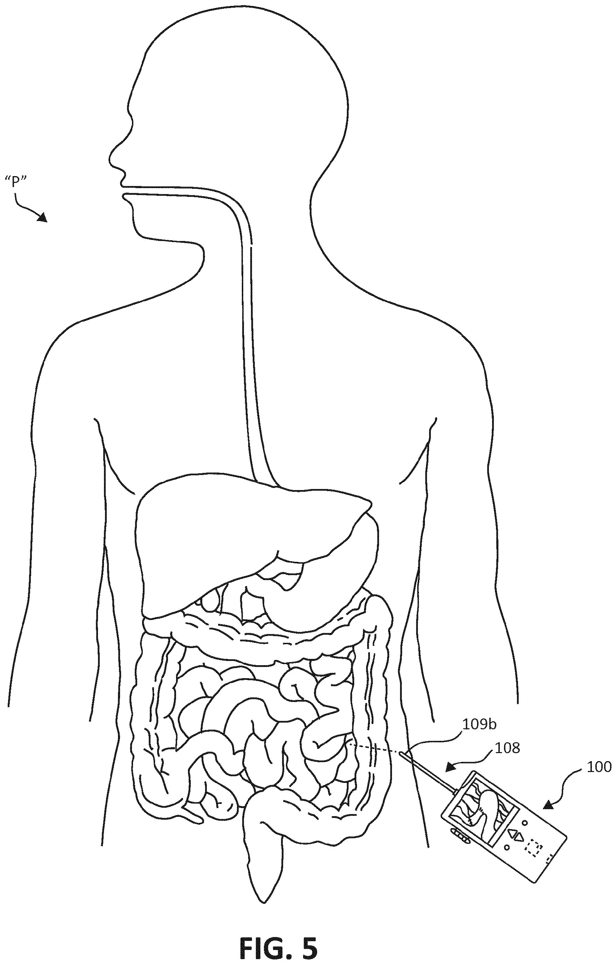

In use, a clinician may perform a post-surgical check-up as a preventative measure, such as, for example, after an anastomotic procedure, as shown in FIG. 5. Probe 108 can be inserted directly into a small incision (or, e.g., through a trocar) made into the abdominal cavity of patient "P" proximate to site of the anastomosis, e.g., to test for anastomotic leaks and/or any infections resulting from the anastomotic leak. Alternatively, probe 108 may be inserted through any natural orifice of the patient "P" (e.g., mouth, nose, anus, etc.). Dial 110 is used to articulate, manipulate, and/or rotate probe 108 to navigate the surgical or wound site. Camera 120 and light source 122 of probe 108 are then used to provide the surgical field of view "SF" of the surgical or wound site wherein the resulting images, video, and/or data are displayed on display 106 in real-time such that the clinician can diagnose and navigate the surgical or wound site using display 106. For example, camera 120 and light source 122 may be used to detect anastomotic leaks at the site of anastomosis, or infections (e.g., pathogens, bacteria, etc.) resulting from the anastomotic leaks. A second incision (not shown) may be made into the patient such that a grasper can be used to facilitate visual inspection of the surgical or wound site.

To enhance visualization of the infection at the surgical or wound site, a UV mode can be selected using controls 103 such that light source 122 of 100 emits UV light. The UV light may be used to illuminate and detect infectious agents not visible under ordinary light. Additionally, the UV light (e.g., UV-C) may be used to treat (e.g., sterilize, disinfect) the surgical or wound site. The surgical or wound site (e.g., the site of infection) may be treated with UV-C light for as long as necessary to adequately sterilize and/or disinfect the surgical or wound site.

Additionally or alternatively, swab 130 may be inserted into recess 124 of probe 108 such that swab 130 can collect tissue samples for testing. For example, after swab 130 is attached to probe 108, dial 110 is actuated to obtain an adequate tissue culture of the surgical or wound site. Additionally or alternatively, a clinician can move (e.g., up, down, left, right, in, out, etc.) device 100 to ensure that an adequate tissue sample is received by swab 130. The swab 130 may then be mixed with or exposed to a reagent and placed within chamber 112a of photometer 112. Photometer 112 and CPU 104 cooperate to provide an RLU reading of the surgical or wound site. The RLU reading and/or other prompts may be displayed on display 106, which may provide a detailed account of the level of infection at the surgical or wound site. If the level of infection at the surgical or wound site exceeds a threshold level, then device 100 will alert a clinician (e.g., through flashing a prompt on display 106, an auditory tone, etc.). Device 100 may then recommend a course of treatment, such as applying UV-C light from light source 122 to the surgical or wound site, performing further surgery, etc.

The images, video, and/or data retrieved by device 100 may be documented and/or recorded into memory 104a of CPU 104. Additionally or alternatively, a user can download the data onto an external device (e.g., tablet, smartphone, computer, etc.) using data interface device 106a.

It should be appreciated that device 100 may be used before, during, and/or after any type of surgical procedure and is not limited to anastomotic procedures. For example, probe 108 of device 100 may be inserted during a procedure and then removed after a monitoring period. The monitoring period may be defined as a timeframe where sufficient time has passed indicating that the post-surgical infection period has passed due to sufficient healing. Device 100 may be used to detect foreign materials (e.g., hernia mesh, sutures, implants, etc.) and infections resulting therefrom. Additionally, device 100 may be used to detect solid or soluble substances (e.g., endotoxins, metabolites) that would indicate a presence of an infection. Device 100 may be configured to perform throat culture tests, blood culture tests, vaginal culture tests, skin and wound culture tests, pap smears, or the like. In embodiments, contrast agents may be applied to the surgical or wound site to, e.g., enhance imaging of the surgical or wound site.

Persons skilled in the art will understand that the structures and methods specifically described herein and shown in the accompanying figures are non-limiting exemplary embodiments, and that the description, disclosure, and figures should be construed merely as exemplary of particular embodiments. It is to be understood, therefore, that the present disclosure is not limited to the precise embodiments described, and that various other changes and modifications may be effected by one skilled in the art without departing from the scope or spirit of the disclosure. Additionally, the elements and features shown or described in connection with certain embodiments may be combined with the elements and features of certain other embodiments without departing from the scope of the present disclosure, and that such modifications and variations are also included within the scope of the present disclosure. Accordingly, the subject matter of the present disclosure is not limited by what has been particularly shown and described. The scope of the invention is defined by the appended claims.

## Claims

1. An infection detection device (100), comprising:
a housing (102) having a display (106) configured to output a level of infection at a surgical site;
a probe (108) having a proximal end portion (109a) attached to the housing and a distal end portion (109b) configured for insertion into a surgical site; and
a detection device (112) integrated along the probe, the detection device configured to measure a level of infection at a surgical site and communicate with the display (106) to alert a user when a predetermined level of infection is detected at the surgical site,
the detection device including a sterile applicator (130) selectively attachable to the distal end portion of the probe, the sterile applicator configured to retrieve a tissue sample from the surgical site **characterized in that** the device further comprises a photometer (112) configured to measure a level of infection from the tissue sample retrieved by the sterile applicator and wherein the photometer includes a reading chamber (112a) configured to receive the sterile applicator for testing a level of infection of the tissue sample therewith.

2. The infection detection device of claim 1, wherein the detection device includes a camera (120) disposed at a distal end portion (109b) of the probe, the camera defining a surgical field of view (SF).

3. The infection detection device of claim 2, wherein the detection device includes a light source (122) disposed at the distal end portion (109b) of the probe, the light source configured to illuminate the surgical field of view (SF) defined by the camera, the light source configured to treat an infection at a surgical site.

4. The infection detection device of claim 1, wherein the detection device includes a computing device including a processor (104) and a memory (104a) configured to perform at least one operation of the infection detection device.

5. The infection detection device of claim 5, wherein the display (106) is configured for displaying at least one output of the computing device.

6. The infection detection device of claim 6, wherein the computing device is configured to determine the predetermined level of infection at the surgical site.

7. The infection detection device of claim 2, wherein the display (106) is configured for displaying the surgical field of view (SF) defined by the camera (120).

8. The infection detection device of any preceding claim, further comprising a dial (110) disposed on the housing (102) and operatively connected to the probe (108), wherein the dial is configured to selectively rotate and articulate the probe relative to the housing.

9. The infection detection device of claim 3, wherein the light source (122) is configured to radiate ultraviolet light to visualize a bacterial infection at a surgical site.

10. The infection detection device of claim 3, wherein the light source (122) is configured to radiate ultraviolet light with a wavelength from about a 260 nanometer to about a 280 nanometer to treat tissue.

11. The infection detection device of claim 4, wherein the distal end portion (109b) of the probe includes a recess (124) configured to receive the sterile applicator (130).

12. The infection detection device of claim 6, further comprising a data interface device (106a) configured to interface with an external device, the data interface device configured to upload data from the computing device onto the external device, the data interface device configured to download data from the external device onto the computing device.

## Patentansprüche

1. Infektionsnachweisvorrichtung (100), die Folgendes umfasst:
ein Gehäuse (102), das eine Anzeige (106) aufweist, die konfiguriert ist, um einen Infektionsgrad an einer chirurgischen Stelle auszugeben;
eine Sonde (108), die einen proximalen Endabschnitt (109a), der an dem Gehäuse befestigt ist, und einen distalen Endabschnitt (109b) aufweist, der zum Einführen in eine chirurgischen Stelle konfiguriert ist; und
eine Nachweisvorrichtung (112), die entlang der Sonde integriert ist, wobei die Nachweisvorrichtung konfiguriert ist, um einen Infektionsgrad an einer chirurgischen Stelle zu messen und mit der Anzeige (106) zu kommunizieren, um einen Benutzer zu warnen, wenn ein zuvor bestimmter Infektionsgrad an der chirurgischen Stelle nachgewiesen wird,
wobei die Nachweisvorrichtung einen sterilen Applikator (130) beinhaltet, der an dem distalen Endabschnitt der Sonde wahlweise befestigbar ist, wobei der sterile Applikator konfiguriert ist, um eine Gewebeprobe von der chirurgischen Stelle zu entnehmen, **dadurch gekennzeichnet, dass** die Vorrichtung ferner ein Photometer (112) umfasst, das konfiguriert ist, um einen Infektionsgrad von der durch den sterilen Applikator entnommenen Gewebeprobe zu messen, und wobei das Photometer eine Lesekammer (112a) beinhaltet, die konfiguriert ist, um den sterilen Applikator zum Testen eines Infektionsgrades der Gewebeprobe damit aufzunehmen.

2. Infektionsnachweisvorrichtung nach Anspruch 1, wobei die Nachweisvorrichtung eine Kamera (120) beinhaltet, die an einem distalen Endabschnitt (109b) der Sonde angeordnet ist, wobei die Kamera ein chirurgisches Sichtfeld (surgical field of view - SF) definiert.

3. Infektionsnachweisvorrichtung nach Anspruch 2, wobei die Nachweisvorrichtung eine Lichtquelle (122) beinhaltet, die an dem distalen Endabschnitt (109b) der Sonde angeordnet ist, wobei die Lichtquelle konfiguriert ist, um das durch die Kamera definierte chirurgische Sichtfeld (SF) zu beleuchten, wobei die Lichtquelle konfiguriert ist, um eine Infektion an einer chirurgischen Stelle zu behandeln.

4. Infektionsnachweisvorrichtung nach Anspruch 1, wobei die Nachweisvorrichtung eine Rechenvorrichtung beinhaltet, die einen Prozessor (104) und einen Speicher (104a) beinhaltet, die konfiguriert ist, um wenigstens einen Ablauf der Infektionsnachweisvorrichtung durchzuführen.

5. Infektionsnachweisvorrichtung nach Anspruch 5, wobei die Anzeige (106) zum Anzeigen wenigstens einer Ausgabe der Rechenvorrichtung konfiguriert ist.

6. Infektionsnachweisvorrichtung nach Anspruch 6, wobei die Rechenvorrichtung konfiguriert ist, um den zuvor bestimmten Infektionsgrad an der chirurgischen Stelle zu bestimmen.

7. Infektionsnachweisvorrichtung nach Anspruch 2, wobei die Anzeige (106) zum Anzeigen des durch die Kamera (120) definierten chirurgischen Sichtfelds (SF) konfiguriert ist.

8. Infektionsnachweisvorrichtung nach einem der vorhergehenden Ansprüche, die ferner ein Einstellrad (110) umfasst, das an dem Gehäuse (102) angeordnet und mit der Sonde (108) wirkgekoppelt ist, wobei das Einstellrad konfiguriert ist, um die Sonde relativ zu dem Gehäuse wahlweise zu drehen und gelenkig anzubringen.

9. Infektionsnachweisvorrichtung nach Anspruch 3, wobei die Lichtquelle (122) konfiguriert ist, um ultraviolettes Licht abzustrahlen, um eine bakterielle Infektion an einer chirurgischen Stelle sichtbar zu machen.

10. Infektionsnachweisvorrichtung nach Anspruch 3, wobei die Lichtquelle (122) konfiguriert ist, um ultraviolettes Licht mit einer Wellenlänge von etwa 260 Nanometer bis etwa 280 Nanometer abzustrahlen, um Gewebe zu behandeln.

11. Infektionsnachweisvorrichtung nach Anspruch 4, wobei der distale Endabschnitt (109b) der Sonde eine Aussparung (124) beinhaltet, die konfiguriert ist, um den sterilen Applikator (130) aufzunehmen.

12. Infektionsnachweisvorrichtung nach Anspruch 6, die ferner eine Datenschnittstellenvorrichtung (106a) umfasst, die konfiguriert ist, um sich über eine Schnittstelle mit einer externen Vorrichtung zu verbinden, wobei die Datenschnittstellenvorrichtung konfiguriert ist, um Daten von der Rechenvorrichtung auf die externe Vorrichtung hochzuladen, wobei die Datenschnittstellenvorrichtung konfiguriert ist, um Daten von der externen Vorrichtung auf die Rechenvorrichtung herunterzuladen.

## Revendications

1. Dispositif de détection d'infection (100), comprenant :
un boîtier (102) ayant un dispositif d'affichage (106) configuré pour émettre un niveau d'infection au niveau d'un site chirurgical ;
une sonde (108) ayant une partie d'extrémité proximale (109a) fixée au boîtier et une partie d'extrémité distale (109b) conçue pour être insérée dans un site chirurgical ; et
un dispositif de détection (112) intégré le long de la sonde, le dispositif de détection étant configuré pour mesurer un niveau d'infection au niveau d'un site chirurgical et communiquer avec le dispositif d'affichage (106) pour alerter un utilisateur lorsqu'un niveau prédéterminé d'infection est détecté au niveau du site chirurgical,
le dispositif de détection comportant un applicateur stérile (130) pouvant être fixé de manière sélective à la partie d'extrémité distale de la sonde, l'applicateur stérile étant conçu pour récupérer un échantillon de tissu du site chirurgical, **caractérisé en ce que** le dispositif comprend en outre un photomètre (112) configuré pour mesurer un niveau d'infection de l'échantillon de tissu récupéré par l'applicateur stérile et le photomètre comportant une chambre de lecture (112a) conçue pour recevoir l'applicateur stérile afin de tester un niveau d'infection de l'échantillon de tissu avec celui-ci.

2. Dispositif de détection d'infection selon la revendication 1, le dispositif de détection comportant une caméra (120) disposée au niveau d'une partie d'extrémité distale (109b) de la sonde, la caméra définissant un champ de vision chirurgical (SF).

3. Dispositif de détection d'infection selon la revendication 2, le dispositif de détection comportant une source de lumière (122) disposée au niveau de la partie d'extrémité distale (109b) de la sonde, la source de lumière étant conçue pour éclairer le champ de vision chirurgical (SF) défini par la caméra, la source lumineuse étant conçue pour traiter une infection sur un site chirurgical.

4. Dispositif de détection d'infection selon la revendication 1, le dispositif de détection comportant un dispositif informatique comportant un processeur (104) et une mémoire (104a) configurée pour effectuer au moins une opération du dispositif de détection d'infection.

5. Dispositif de détection d'infection selon la revendication 5, le dispositif d'affichage (106) étant configuré pour afficher au moins une sortie du dispositif informatique.

6. Dispositif de détection d'infection selon la revendication 6, le dispositif informatique étant configuré pour déterminer le niveau prédéterminé d'infection au niveau du site chirurgical.

7. Dispositif de détection d'infection selon la revendication 2, le dispositif d'affichage (106) étant configuré pour afficher le champ de vision chirurgical (SF) défini par la caméra (120).

8. Dispositif de détection d'infection selon l'une quelconque des revendications précédentes, comprenant en outre un cadran (110) disposé sur le boîtier (102) et relié fonctionnellement à la sonde (108), le cadran étant conçu pour faire tourner et articuler sélectivement la sonde par rapport au boîtier de sonde.

9. Dispositif de détection d'infection selon la revendication 3, la source de lumière (122) étant conçue pour rayonner de la lumière ultraviolette afin de visualiser une infection bactérienne au niveau d'un site chirurgical.

10. Dispositif de détection d'infection selon la revendication 3, la source de lumière (122) étant configurée pour rayonner de la lumière ultraviolette avec une longueur d'onde d'environ 260 nanomètres à environ 280 nanomètres pour traiter du tissu.

11. Dispositif de détection d'infection selon la revendication 4, la partie d'extrémité distale (109b) de la sonde comportant un évidement (124) conçu pour recevoir l'applicateur stérile (130).

12. Dispositif de détection d'infection selon la revendication 6, comprenant en outre un dispositif d'interface de données (106a) configuré pour s'interfacer avec un dispositif externe, le dispositif d'interface de données étant configuré pour télécharger des données du dispositif informatique sur le dispositif externe, le dispositif d'interface de données étant configuré pour télécharger des données à partir du dispositif externe sur le dispositif informatique.
